# EUROPEAN PATENT APPLICATION

(11) **EP 1 213 301 A2**
(43) Date of publication of application: **12.06.2002**
(21) Application number: 01310310.6
(22) Date of filing: 10.12.2001
(51) Int. Cl.: C07K 16/00, C07K 16/18, C12N 5/20, C07K 14/435, C07K 7/08, G01N 33/68

(54) **Anti-abnormal type prion monoclonal antibody, process for producing the same, and immunoassay using the same**

(30) Priority: 08.12.2000 JP 2000374145
(71) Applicant: FUJIREBIO INC., Tokyo 103-0007 (JP)
(72) Inventor: Kurano, Yoshihiro, c/o Fujirebio Inc., Tokyo 103-0007 (JP); Umetani, Atsushi, c/o Fujirebio Inc., Tokyo 103-0007 (JP); Miyakoshi, Hideo, c/o Fujirebio Inc., Tokyo 103-0007 (JP); Yanagiya, Takayuki, c/o Fujirebio Inc., Tokyo 103-0007 (JP)
(74) Representative: Denison, Christopher Marcus

(57) **Abstract**

A monoclonal antibody which enables to distinguish the abnormal type prion from the normal type prion, as well as production process thereof, is disclosed. The anti-abnormal type prion monoclonal antibody of the invention reacts with abnormal type prion by antigen-antibody reaction but does not substantially react with normal type prion by antigen-antibody reaction. The anti-abnormal type prion monoclonal antibody of the invention may be obtained by immunizing an animal with an immunogen including a peptide containing a plurality of regions in the abnormal type prion, which regions are discontinuous each other in primary amino acid sequence of the abnormal type prion.

## Description

### I.

The present invention relates to an anti-abnormal type prion monoclonal antibody, process for producing the same, and immunoassay of abnormal type prion using the same.

### II.

Cranial nervous diseases including Creutzfeldt-Jakob disease (hereinafter referred to as "CJD" for short), serapie disease of sheep, transmissible encephalopathy of mink and the like develop after a long incubation period. They are mainly characterized by spongiform change of brain tissue and amyloid spot (kuru spot), that are almost localized in nerve system, and progressively aggravate to death. Although the cause of the diseases has not been fully clarified, the so called "prion hypothesis" which assumes that the diseases are not caused by infectious pathogen such as a virus, but are caused by deposition of abnormal type prion protein, is now believed by most of the researchers. These diseases are diagnosed by pathological analysis of thin section of brain tissue.

It is thought that these diseases are infectious, and infection is made by eating cranial nerve tissue (e.g., kuru disease and the like), or by medical treatment such as piercing of electrodes or transplantation of endocranium. Recently, it is thought that bovine spongiform encephalopathy and new type CJD are infected by oral infection.

Normal prion protein is a glycoprotein existing in cell membranes, which widely occurs in various eukaryotes such as yeasts. The gene encoding normal prion is a single gene and the encoded amino acid sequence is very well conserved among the mammals. Especially, it has been reported that the homology of the amino acid sequences among human, sheep and bovine is not less than about 90%.

Although the function of the normal type prion protein has not yet been clarified, since the amino acid sequence is well conserved, it is presumed that it plays an important role in generation, development and function of nerve tissue. With a knock out mouse in which the prion gene is knocked out, abnormal walking such as shaking of the lower half of the body with aging, and pathologically, atrophy of cerebellum, especially deciduation of cerebellar Purkinje cells, is observed.

In human, although variations in a part of the amino acid sequence (primary structure) of the prion protein among individuals have been reported, there is no difference between the amino acid sequences of prion protein in CJD patients and normal individuals. Therefore, it is thought that deposition of the abnormal prion protein is not because of the amino acid sequence, but because of the difference in stereostructure. Therefore, the conventional anti-prion antibodies which recognize the primary amino acid sequence of prion cannot distinguish the abnormal type prion from the normal type prion. Further, since the amino acid sequence is well conserved between animals, it is presumed that antigenicity of prion is low. Thus, production of an anti-abnormal type prion antibody using an immunogen keeping the stereostructure thereof, in which the antigenecity of the immunogen is increased, is demanded.

An object of the present invention is to provide a monoclonal antibody which can distinguish the abnormal type prion from the normal type prion, as well as a production process thereof. Another object of the present invention is to provide an immunoassay of the abnormal type prion using the monoclonal antibody.

It is thought that a monoclonal antibody which can distinguish the abnormal type prion from the normal type prion may be obtained by immunizing an animal with the abnormal type prion, obtaining monoclonal antibodies by a conventional method, and by screening a monoclonal antibody which reacts with the abnormal type prion but does not react with the normal type prion. However, not only because of the fact that there are no differences in the amino acid sequence of the normal and abnormal types, but also because of the low species specificity, it is difficult to induce an antibody, especially an antibody which can distinguish the abnormal type prion from the normal type prion. Further, abnormal type prion is a protein which is difficult to obtain in a sufficient amount for use as an immunogen. The frequency of the diseases yielding abnormal type prion is low, and the facilities which can deal with the abnormal type prion are limited because it is a strong pathogen. Because of these, it is difficult to obtain the abnormal type prion in a large amount. Further, to use the abnormal type prion protein as an immunogen, it is necessary to purify the protein to some degree. However, the protein is insolubilized in the purification step, and the insolubilized protein is not suitable as an immunogen. To use as an immunogen, the protein is solubilized, e.g. using a detergent, surfactant or chaotropic agent. However, it is difficult to ensure that the stereostructure unique to the abnormal type be kept during the solubilization step. Because of these reasons, it is difficult to prepare a monoclonal antibody which can distinguish the abnormal type prion from the normal type prion.

The present inventors intensively studied to locate the exposed regions in the stereostructure of the abnormal type prion, and to succeed in preparing a monoclonal antibody which can distinguish the abnormal type prion from the normal type prion, thereby completing the present invention.

That is, the present invention provides an anti-abnormal type prion monoclonal antibody which reacts with abnormal type prion but does not substantially react with normal type prion by antigen-antibody reaction, or an antigen-binding fragment thereof. The present invention also provides a hybridoma which produces the monoclonal antibody according to the present invention. The present invention further provides a method for measuring abnormal type prion by an immunoassay utilizing the antigen-antibody reaction between the monoclonal antibody according to the present invention and an abnormal type prion. The present invention still further provides an immunoassay kit for carrying out the immunoassay of the present invention, comprising the monoclonal antibody or the antigen-binding fragment thereof according to the present invention. The present invention still further provides process for producing the anti-abnormal type prion monoclonal antibody according to the present invention, comprising immunizing an animal with an immunogen including a peptide containing a plurality of regions in said abnormal type prion, which regions are discontinuous each other in primary amino acid sequence of said abnormal type prion; preparing hybridomas originated from antibody-producing cells of the immunized animal; screening a hybridoma which produces an anti-abnormal type prion monoclonal antibody which reacts with the abnormal type prion but does not substantially react with the normal type prion by antigen-antibody reaction; and recovering said anti-abnormal type prion monoclonal antibody from the hybridoma selected by the screening. The present invention still further provides an immunogen used in the above-mentioned process for producing the monoclonal antibody of the present invention.

By the present invention, an anti-abnormal type prion monoclonal antibody which reacts with abnormal type prion but does not substantially react with normal type prion by antigen-antibody reaction, or an antigen-binding fragment thereof was first provided. As a result, immunoassay of the abnormal type prion was first attained. Thus, the present invention will make a great contribution to the diagnosis of bovine and other animals infected with bovine spongiform encephalopathy and the diagnosis of CJD.

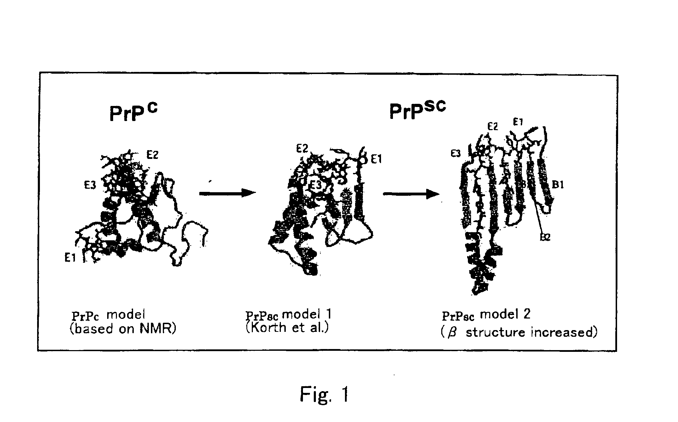

Fig. 1 schematically shows the stereostructure of the abnormal type prion described in Korth et al., and the stereostructure of the abnormal type prion, which was presumed by the present inventors.

The monoclonal antibody according to the present invention reacts with the abnormal type prion by antigen-antibody reaction but does not substantially reacts with the normal type prion. The term "does not substantially react" means that the immunological reactivity with the normal type prion is lower than the immunological reactivity with the abnormal type prion to a discemable degree. Thus, even if a monoclonal antibody has a cross-reactivity with the normal type prion, in case where the affinity to the normal type prion is lower than the affinity to the abnormal type prion to a discernable degree, the monoclonal antibody is included within the definition of "does not substantially react with the normal type prion", and is included in the scope of the present invention. Needless to say, a monoclonal antibody which does not have a cross-reactivity with the normal type prion, that is, a monoclonal antibody which reacts with the abnormal type prion but does not react with the normal type prion is preferred.

The monoclonal antibody of the present invention is preferably one which reacts with the abnormal type prion but does not react with the normal type prion in immunohistostaining. In the conventional immunohistostaining for staining prion, the so called "acid-autoclave treatment" (i.e., the tissue is immersed in HCl solution with a concentration of 1.0 to 100 mM and then autoclaved at 121°C for 20 minutes) is performed and then the immunohistostaining is carried out. The monoclonal antibody according to the present invention is preferably one which reacts with the abnormal type prion existing in the tissue which was not subjected to a pretreatment such as the acid-autoclave treatment. Examples of such an anti-abnormal type prion monoclonal antibody include the monoclonal antibody produced by hybridoma EBEB4C3Ebb. The hybridoma EBEB4C3Ebb has been deposited with National Institute of Advanced Industrial Science and Technology (formerly National Institute of Bioscience and Human-Technology, Agency of Industrial Science and Technology) at AIST Tsukuba Central 6, 1-1, Higashi 1-chome, Tsukuba-shi, Ibaraki-ken 305-8566 Japan as of September 1, 2000 under the accession No. FERM P-18013 (original deposition), and the deposition was converted to international deposition under the Budapest Treaty on November 21, 2001, the accession No. of the international deposition being FERM BP-7808.

The present invention also provides antigen-binding fragments of the above-described monoclonal antibody according to the present invention. The term "antigen-binding fragment" herein means fragment such as Fab fragment or F(ab')₂ fragment of the antibody, which exhibits antigen-binding property of the antibody. These fragments may easily be obtained by cleaving the monoclonal antibody of the present invention with papain or pepsin according to a conventional method. These antigen-antibody fragments may be used in the immunoassays described below equally as the monoclonal antibody of the present invention

As mentioned above, it is difficult to prepare a monoclonal antibody which reacts with the abnormal type prion but does not substantially react with the normal type prion by using the abnormal type prion as an immunogen. To solve this problem, the present inventors originally presumed the stereostructure of the abnormal type prion. Fig. 1 shows the stereostructure of the normal prion (PrP^{C} model), the stereostructure of the abnormal type prion presumed by Korth et al. (PrP^{SC} model 1, C. Korth et al., Nature 390:74-77, 1997), and the stereostructure of the abnormal type prion (PrP^{SC} model 2) presumed by the present inventors. The stereostructure of the abnormal type prion, which was presumed by the present inventors, contains more β sheet structures than in the stereostructure presumed by Korth et al. The grounds of this presumption are as follows: From the results of CD spectrum and FT-IR, it is said that during the process of changing from the normal type to the abnormal type, α helix structure is decreased from 40% to 30%, and β structure is increased from very little to 45%. According to the calculation based on the stereostructure (123-231a.a.) by NMR, except for the very flexible repeating sequence at the N-terminal of which structure cannot be determined, the secondary structure of the normal type contains 25% of α helix and 3.4% of β structure (assuming that the full length is 231 amino acid residues). Assuming that the contribution to the content of α helix structure by the N-terminal region which cannot be included in the calculation is about 15%, according to the model of Korth et al., in the abnormal type, α helix is 36% and β sheet is 7%. Therefore, the model by Korth et al. only accounts for a part of the results of CD spectrum and FT-IR. In view of this, the present inventors thought a structure in which α helix is decreased and β structure is increased. According to the prediction of secondary structure by SST, the contents of the structures in the secondary structure are: α helix 5.6%, and β structure 18%. Aside from the precision of the prediction of the secondary structure, prion protein is a protein which likely adopts β structure rather than α helix. Looking into more detail, about the half of the α helix 2 of the normal type is judged as β structure and the other half is C structure (random coil). In α helix 3, except for the residues which are judged as constituting α helix in the latter half thereof, the other regions are in β structure or C structure. Thus, the present inventors made a model that the former half of α helix 2 and the latter half of α helix 3 changed into β structure (model 2).

In this model, unlike the model by Korth et al., all of the E1, E2 and E3 regions constituting the epitope of 15B3 are structurally changed. However, in the molecule model, all of the 3 regions can be gathered by assigning β hairpin structure to these regions, so that there is no contradiction. Further, the contents of the secondary structure of this model are: α helix 27%, β structure 17%. Although these contents do not completely agree with the results of CD spectrum and FT-IR, they are closer to the results than those in the model by Korth et al.

The present inventors thought that a monoclonal antibody which specifically reacts with the abnormal type prion may be obtained by using a peptide as an immunogen, which peptide consists essentially of the ligation of at least two regions each of which is exposed to the outside, that is, at least two regions selected from the group consisting of B region (the 128th to 131st amino acid in the primary amino acid sequence of prion, hereinafter indicated as "128-131a.a.", other regions being indicated in the same way), B2 region (138-141a.a.), B3 region (149-152 a.a.), E1 region (141-149 a.a.) and E2 region (164-170a.a.) and E3 region (214-231 a.a.). As described in Examples below in detail, by using composite immunogen comprising a peptide (SEQ ID NO:1) consisting of E1 region ligated with B2 and B3 regions, and a peptide (SEQ ID NO:2) which comprises the E2 region, which peptides are bound to the same KLH molecule, the monoclonal antibody produced by the above-mentioned hybridoma EBEB4C3Ebb was obtained. The above-described regions may be directly ligated or indirectly ligated by inserting one to several amino acids therebetween. Thus, the term "are ligated" includes both the cases where the regions are directly ligated and the cases where the regions are indirectly ligated via one to several amino acid residues. Further, in each of the above-described regions, one to several amino acid residues may be deleted or may be added. The peptides consisting essentially of the ligation of these regions can easily be prepared by using a commercially available automatic peptide synthesizer. The idea of using peptide in which a plurality of discontinuous regions in the primary amino acid sequence of a protein are ligated as an immunogen was originally created by the present inventors. The amino acid sequences of the above-mentioned regions are as follows:
B1: Tyr Met Leu Gly
B2: Ile Ile His Phe
B3: Tyr Tyr Arg Glu
E1: Gly Ser Asp Tyr Glu Asp Arg
E2: Arg Pro Met Asp Glu Tyr Ser
E3: Cys Ile Thr Gln Tyr Glu Arg Glu Ser Gin Ala Tyr Tyr Gln Arg Gly Ser Ser
The invention also encompasses the use of immunogens based on the corresponding homologous regions of prionproteins from other species, especially human or sheep. Similarly, antibodies raised using such immunogens and hybridomas producing such antibodies are also encompassed by the invention, as are corresponding methods, kits and processes.

Although the above-described peptide may be used as it is as an immunogen, it is preferred to bind the peptide to a carrier molecule such as keyhole limpet hemocyanin (KLH) or bovine serum albumin (BSA) because the antigenecity of the immunogen is increased. Further, it is preferred to use an immunogen in which a plurality of kinds of the above-described peptide are bound to a single carrier molecule.

The monoclonal antibody according to the present invention may be prepared by a conventional method except that the above-described immunogen is used. That is, an animal is immunized with the above-described immunogen, and hybridomas derived from antibody-producing cells of the immunized animal are prepared. The hybridomas are screened for those producing monoclonal antibodies which react with the abnormal type prion and does not substantially react with the normal type prion, and the desired monoclonal antibody is recovered from the screened hybridoma. The method for preparing hybridomas by fusing antibody-producing cells such as spleen cells and lymphocytes with immortalized cells such as myeloma cells is well-known in the art.

By an immunoassay utilizing the antigen-antibody reaction between the monoclonal antibody according to the present invention and the abnormal type prion, the abnormal type prion may be measured. The term "measure" herein includes both detection and quantitation. The immunoassays *per se* are well-known in the art, and any of the well-known immunoassays may be employed in the present invention. That is, classifying the known immunoassays according to the reaction type, known immunoassays include sandwich immunoassays, competition immunoassays and agglutination immunoassays. Classifying the known immunoassays according to the label employed, known immunoassays include enzyme immunoassays, radio immunoassays, fluorescence immunoassays and the like. Any of these immunoassays are included in the "immunoassay" defined in the present invention. Further, immunohistostaining, Western blotting and the like are also included in the "immunoassay" defined in the present invention.

These immunoassays *per se* are well-known in the art, and so it is not necessary to explain these immunoassays in the present specification. Briefly, in sandwich immunoassays, for example, the monoclonal antibody or an antigen-binding fragment thereof is immobilized on a solid support as a first antibody. The first antibody is then reacted with a sample, and after washing the solid support, the resultant is then reacted with a second antibody which reacts with the abnormal type prion by antigen-antibody reaction (the second antibody may be an antibody which also reacts with the normal type prion, and may be either a monoclonal antibody or a polyclonal antibody). After washing the solid support, the second antibody bound to the solid support is measured. By labeling the second antibody with an enzyme, fluorescent substance, radioactive substance, biotin or the like, measurement of the second antibody may be attained by measuring the label. The above-mentioned measurement is conducted for a plurality of standard samples each containing a known concentration of the abnormal type prion, and the relationship between the concentrations of the abnormal type prion in the standard samples and the measured amounts of the label is plotted to prepare a calibration curve. The abnormal type prion in a test sample may be determined by applying the measured amount to the calibration curve. It should be noted that the above-mentioned first antibody and the above-mentioned second antibody may be exchanged. In agglutination immunoassays, the monoclonal antibody according to the present invention or an antigen-binding fragment thereof is immobilized on particles such as latex particles, and the particles are reacted with a sample, followed by measurement of the absorbance. The above-mentioned measurement is conducted for a plurality of standard samples each containing a known concentration of the abnormal type prion, and the relationship between the concentrations of the abnormal type prion in the standard samples and the measured absorbance is plotted to prepare a calibration curve. The abnormal type prion in a test sample may be determined by applying the measured absorbance to the calibration curve.

The reagents necessary for each type of immunoassay are also well-known in the art. Except for the monoclonal antibody used, the immunoassay according to the present invention may be carried out using an ordinary kit for immunoassay. For example, such an immunoassay kit may usually include buffer solution, solid support, labeled second antibody and the like.

The method of the present invention will now be described in more detail by way of examples thereof. It should be noted that the present invention is not restricted to the examples below.

### (1) Preparation of Immunogen

Korth et al. analyzed the epitope of the bovine prion protein by using a recombinant antigen expressed by a genetic recombination technique, and reported that the major antigenic regions of the prion protein are the above-mentioned three regions, i.e., E1, E2 and E3 regions (Korth et al., *supra).* In the stereostructure of the normal type prion expected from these results, E2 and E3 regions are close, but E1 region is apart from E2 and E3 regions. In contrast, based on the above-mentioned assumption wherein more β structures are included, two β chains (B2 and B3) are newly formed by assuming that E1 has a loop structure, so that E1 gives a new epitope. Thus, E1B1 peptide (SEQ ID NO:1) consisting of ligation of B2, E1 and B3 regions, and E2-1 peptide (SEQ ID NO:2) comprising E2 region were chemically synthesized by a conventional method. These peptides were bound to KLH (i.e., both of these peptides are bound to a single KLH molecule), and the obtained conjugates were used as the immunogen.

### (2) Immunization and Cell Fusion

The immunogen with a concentration of 0.5 to 1.0 mg/mL was suspended in an equivolume of Freund's complete adjuvant, and BALB/C mice were immunized with the resulting immunogen suspension twice or three times at a dose of 0.2 - 0.3 ml/mouse per immunization. Three or four days after the final immunization, the spleen was recovered from each mouse and the cells were dispersed. The cells were fused with P3U1 myeloma cells by polyethylene glycol method.

Hybridomas were selected by culture in HAT medium, and the screening of the antibody-producing cells was carried out by ELISA using an antigen prepared by binding the above-described synthetic peptide bound to bovine serum albumin (BSA). That is, each cell culture supernatant was placed in a well of an ELISA plate (96 well-type) in which the above-mentioned BSA conjugate was immobilized. After reaction and washing, horse radish peroxidase (HRP)-labeled anti-mouse Ig (IgG + IgM) was reacted and existence of antibody-producing cell was checked based on the coloring reaction. The number of positive clones of hybridoma is shown in Table 1.

**Table 1**

| | |
|---|---|
| Fusion Efficiency | 100% |
| Rate of Hybridomas Producing IgG | 100% |
| Number of Positive Wells in Primary Screening | 114 wells |
| Number of Positive Wells in Secondary Screening | 54 wells |

### (3) Screening of Monoclonal Antibodies Which Specifically Reacts with Abnormal Type Prion.

For the purpose of confirming specificities of the antibodies, brain thin sections were prepared from the brain of a patient suffering from GSS (Gerstmann Straussler syndrome), and the reactivities of the antibodies were studied by immunohistostaining screening. As the specimens, both of the specimens subjected to the acid-autoclave treatment and not subjected to this pretreatment were used, and antibody clones which specifically reacts with the abnormal type prion protein were selected. As a control, thin sections of brain from normal human, which were subjected to the acid-autoclave treatment or not subjected to this pretreatment, were subjected to the above-described immunohistostaining, thereby confirming that no reactions were observed. The concrete operations of this immunohistostaining were as follows:
1. Tissue fragment was deparaffinized with xylene and the alcohol concentration was sequentially decreased to hydrate the tissue fragment.
2. The resultant was washed with distilled water for 5 minutes.
3. The resultant was immersed in 0.3% hydrogen peroxide/methanol solution for 30 minutes to remove endogenous peroxidase.
4. The resultant was washed with physiological phosphate buffer for 5 minutes.
5. The tissue to be subjected to the acid-autoclave treatment was immersed in 10 to 1000 mM hydrochloric acid solution at 121°C for 20 minutes.
6. Rabbit serum diluted to 5% with physiological phosphate buffer was reacted with the tissue sample at room temperature for 30 minutes.
7. Excess serum was removed and supernatant of the culture medium of hybridomas was reacted with the tissue sample at room temperature for 1 hour.
8. The resultant was washed with physiological phosphate buffer for 5 minutes.
9. Diluted biotinylated anti-mouse immunoglobulin rabbit antibody was reacted at room temperature for 1 hour.
10. The resultant was washed with physiological phosphate buffer for 5 minutes.
11. The resultant was reacted with diluted ABC reagent (commercially available from Vector) at room temperature for 30 minutes.
12. The resultant was washed with physiological phosphate buffer for 5 minutes.
13. The resultant was reacted with a solution of the substrate of peroxidase at room temperature for 5 to 30 minutes.
14. The resultant was washed with distilled water.
15. The resultant was subjected to counter staining with Lillie-Mayer hematoxylin, followed by washing and mounted.

The hybridomas selected by the above-described operations are shown in Table 2.

**Table 2**

| | GSS | | Normal tissue | | | | | |
|---|---|---|---|---|---|---|---|---|
| | | | cerebellum | | cerebrum | | thalamus | |
| Autoclave | + | - | + | - | + | - | + | - |
| EB22D1-2 | + | - | - | - | - | - | - | - |
| EB12D21 | +^{w} | - | - | - | - | - | - | - |
| EBEB4C3Ebb | + | + | - | - | - | - | - | - |
| EB12A32 | +^{w} | +^{w} | - | - | - | - | - | - |
| EB22B61 | + | + | - | - | - | - | - | - |

(In Table 2, in the line of "Autoclave", the symbol "+" means that the acid-autoclave treatment was performed, and the symbol "-" means that the acid-autoclave treatment was not performed. In the line of each monoclonal antibody, the symbol "+" means positive, the symbol "-" means negative, and the symbol "+^{w}" means weakly positive.)

### (4) Results

As shown, monoclonal antibodies which react with the abnormal type prion but do not react with the normal type prion, which enable to distinguish the abnormal type prion from the normal type prion, were obtained. The hybridoma EBEB4C3Ebb which produces such a monoclonal antibody has been deposited with National Institute of Advanced Industrial Science and Technology under the Budapest Treaty under an accession No. FERM BP-7808, as mentioned above.

## Claims

1. An anti-abnormal type prion monoclonal antibody which reacts with abnormal type prion but does not substantially react with normal type prion by antigen-antibody reaction, or an antigen-binding fragment thereof.

2. The monoclonal antibody or the antigen-binding fragment thereof according to claim 1, which reacts with said abnormal type prion and does not substantially react with said normal type prion in immunohistostaining.

3. The monoclonal antibody or the antigen-binding fragment thereof according to claim 1 or 2, which reacts with said abnormal type prion which was not subjected to pretreatment.

4. The monoclonal antibody or the antigen-binding fragment thereof according to any one of claims 1 to 3, originated from an animal immunized with an immunogen comprising a carrier and a peptide having the amino acid sequence shown in SEQ ID NO: 2.

5. A monoclonal antibody or the antigen-binding fragment thereof, which is produced by hybridoma EBEB4C3Ebb (FERM BP-7808).

6. The monoclonal antibody or the antigen-binding fragment thereof according to any one of claims 1 to 5, which is a monoclonal antibody.

7. A hybridoma which produces the monoclonal antibody according to any one of claims 1 to 5.

8. A method for measuring abnormal type prion by an immunoassay utilizing said antigen-antibody reaction between said monoclonal antibody according to any one of claims 1 to 6, and an abnormal type prion.

9. An immunoassay kit for carrying out the method of claim 8, comprising the monoclonal antibody or the antigen-binding fragment thereof according to any one of claims 1 to 6.

10. A process for producing the anti-abnormal type prion monoclonal antibody according to any one of claims 1 to 6, comprising immunizing an animal with an immunogen including a peptide consisting essentially of a plurality of regions in said abnormal type prion, which regions are discontinuous each other in primary amino acid sequence of said abnormal type prion, and which regions are ligated each other in said peptide; preparing hybridomas originated from antibody-producing cells of the immunized animal; screening a hybridoma which produces an anti-abnormal type prion monoclonal antibody which reacts with said abnormal type prion by antigen-antibody reaction but does not substantially react with said normal type prion by antigen-antibody reaction; and recovering said anti-abnormal type prion monoclonal antibody from said hybridoma selected by said screening.

11. The process according to claim 10, wherein said immunogen comprises a carrier and said peptide immobilized on said carrier.

12. The process according to claim 11, wherein said immunogen comprises said carrier and a plurality of kinds of said peptide.

13. The process according to any one of claims 10 to 12, wherein said peptide comprises a region containing at least two regions selected from the group consisting of E1 region, E2 region, B1 region, B2 region and B3 region.

14. The process according to claim 13, wherein said peptide has an amino acid sequence shown in SEQ ID NO:1.

15. The process according to claim 14, wherein said immunogen comprises a peptide having the amino acid sequence shown in SEQ ID NO:1 and a peptide having the amino acid sequence shown in SEQ ID NO:2.

16. The anti-abnormal type prion monoclonal antibody as producible by the process according to any one of claims 10 to 15.

17. The immunogen as defined in any one of claims 10 to 15.


SEQUENCE LISTING
<110> FUJIREBIO INC.
<120> Anti-abnormal type prion monoclonal antibody, process for
producing the same and immunoassay using the same
<130> 01PF231
<160> 7

<210> 1
<211> 15
<212> PRT
<213> Artificial Sequence
<220>
<223> Peptide used as immunogen for raising anti-abnormal prion
monoclonal antibody
<400> 1
Ile Ile His Phe Gly Ser Asp Tyr Glu Asp Arg Tyr Tyr Arg Glu
1               5                   10                  15

<210> 2
<211> 12
<212> PRT
<213> Artificial Sequence
<220>
<223> Peptide used as immunogen for raising anti-abnormal prion
monoclonal antibody
<400> 2
Val Tyr Tyr Arg Pro Met Asp Glu Tyr Ser Asn Cys

1                   5                   10

<210>  3

<211>  4

<212>  PRT

<213>  Homo sapience

<400>  3

Tyr Met Leu Gly

1

<210>  4

<211>  4

<212>  PRT

<213>  Homo sapience

<400>  4

Ile Ile His Phe

1

<210>  5

<211>  4

<212>  PRT

<213>  Homo sapience

<400>  5

Tyr Tyr Arg Glu

1

<210>  6

<211>  7

<212>  PRT

<213>  Homo sapience

<400>  6

Gly Ser Asp Tyr Glu Asp Arg

  1               5

<210>  7

<211>  7

<212>  PRT

<213>  Homo sapience

<400>  7

Arg Pro Met Asp Glu Tyr Ser

  1               5